# EUROPÄISCHE PATENTSCHRIFT

(11) **EP 0 649 641 B1**
(45) Veröffentlichungstag und Bekanntmachung des Hinweises auf die Patenterteilung: **15.09.1999**
(21) Anmeldenummer: 94115991.5
(22) Anmeldetag: 11.10.1994
(51) Int. Cl.: A61F 2/34

(54) **Konische Hüftgelenkpfanne**
Conical hip joint acetabular cup
Coque acétabulaire conique pour l'articulation de la hanche

(30) Priorität: 21.10.1993 DE 4335931
(43) Veröffentlichungstag der Anmeldung: 26.04.1995
(73) Patentinhaber: CERASIV GmbH INNOVATIVES KERAMIK-ENGINEERING, D-73207 Plochingen (DE)
(72) Erfinder: Pfaff, Hans-Georg, Dipl.-Ing., D-73160 Ostfildern (DE); Kälberer, Hartmut, Dipl.-Ing., D-73779 Deizisau (DE)
(74) Vertreter: Schulz, Wilfried, Dipl.-Ing.

(56) Entgegenhaltungen:
- EP-A- 0 083 708
- EP-A- 0 142 759
- EP-A- 0 162 005
- EP-A- 0 482 320
- CH-A- 677 072
- FR-A- 2 598 609
- FR-A- 2 628 315
- FR-A- 2 682 588
- US-A- 4 180 873

## Beschreibung

Die Erfindung betrifft eine Hüftgelenkpfanne.

Hüftgelenk-Endoprothesen bestehen aus einer Hüftgelenkpfanne, die im Beckenknochen verankert ist und aus einer Kugel, die in die Pfanne drehbar eingesetzt ist und mit einem Schaft im Oberschenkelknochen verankert ist.

Hüftgelenkpfannen bestehen aus einer äußeren Metallschale, welche die Implantataußenkontur darstellt und aus einer inneren Gleitschale, die aus Keramik oder aus Kunststoff (UHMWPE = Ultra High Molekular Weight Polyethylen) hergestellt ist.

Es ist Stand der Technik, die innere Gleitschale in der Metallschale mit Hilfe einer konischen Klemmung zu fixieren. Der Winkel der konischen Klemmung liegt dabei bei 5° 43', d.h. einem Winkelverhältnis von 1:10.

Nachteilig hieran ist, daß die Gleitschale sich beim Einsetzen in die Metallschale leicht verkantet. Dadurch entsteht eine ungleichmäßige Kräfteverteilung, die unter Umständen zum Bruch der Gleitschale führen kann, insbesondere, wenn sie aus Keramik hergestellt ist.

Ein weiterer wesentlicher Nachteil ist, daß nach dem Einfügen der Gleitschale bzw. des Pfanneneinsatzes aufgrund der hohen Klemmkräfte die Gleitschale nicht mehr zerstörungsfrei entfernt Werden kann. Die ist jedoch für den Operateur äußerst wichtig.

Ein weiterer Nachteil ist, daß durch die konstruktive Gestaltung der Gleitschale bzw. des Pfanneneinsatzes mit einer 1:10-Klemmung die Baugröße des gesamten Implantats relativ groß ausgelegt werden muß. Dies ist aus medizinischer Sicht ein Nachteil, weil entweder ein hoher Knochenverlust damit verbunden ist oder - bei dünnen Knochenwandstärken - ein solches Implantat nicht implantiert werden kann.

Aus der nächstliegenden EP-A1-0 083 708 ist eine Hüftgelenkpfanne zum Einsetzen in Knochengewebe mit einer äußeren geschlitzten Metallschale und einer inneren Gleitschale bekannt, wobei die Gleitschale in der Metallschale mit Hilfe einer konischen Klemmung fixiert ist. Die Gleitschale kann aus Keramik hergestellt sein, wobei der Winkel der konischen Klemmung bei 10° liegt.

Der Erfindung liegt die Aufgabe zugrunde, eine Hüftgelenkpfanne zum Einsetzen in Knochengewebe derart zu verbessern, daß der Pfanneneinsatz bzw. die Gleitschale gegen Verdrehen und Herausfallen geschützt ist und sich zerstörungsfrei aus seinem Sitz herausdrücken und auswechseln läßt.

Erfindungsgemäß wird diese Aufgabe durch die Merkmale des Anspruchs 1 gelöst.

Durch die erfindungsgemäße Wahl des Winkels der konischen Klemmung ist die Gleitschale bzw. der Pfanneneinsatz gegen Verdrehen und Herausfallen geschützt. Der besondere Vorteil liegt jedoch darin, daß die Auspreßkräfte der Gleitschale aus der Metallschale wesentlich geringer als im Stand der Technik sind. Bei dem bevorzugten Winkel von 18° der konischen Klemmung betragen die Auspreßkräfte ungefähr nur noch die Hälfte der Einpreßkräfte. Hierdurch ist das Losbrechmoment geringer.

Bei gleicher Wandstärke ist die kraftübertragende Fläche größer als bei der üblichen 1:10-Klemmung. Dadurch hat die Gleitschale bzw. der Pfanneneinsatz eine höhere mechanische Festigkeit oder kann bei gleicher Festigkeit kleiner und flacher gebaut werden. Hierdurch wird der medizinischen Forderung nach kleinen Implantaten Rechnung getragen.

Durch den bevorzugten Außenwinkel von 18° werden somit mehrere Vorteile erzielt.

Zum Heraushebeln der Gleitschale ist erfindungsgemäß auf der Kontaktfläche zwischen Metallschale und Gleitschale zumindest eine Ausnehmung angeordnet. Zweckmäßigerweise sind zwei diametral gegenüberliegende Ausnehmungen angeordnet. Zum Heraushebeln wird ein Ausdrückwerkzeug mit der Form z.B. eines Golfschlägers in die Ausnehmung eingeführt und durch Drehen des Ausdrückwerkzeuges eine Kraft von unten auf die Gleitschale ausgeübt, die sich dadurch leicht von ihrem Preßsitz lösen läßt. Vereinfacht ist das Heraushebeln, wenn zwei Ausdrückwerkzeuge in zwei diametral gegenüberliegende Ausnehmungen eingeführt werden und gleichzeitig verdreht werden, so daß die Gleitschale gleichzeitig von zwei Seiten von unten in Auspreßrichtung belastet wird.

Die geringeren Losbrechmomente ermöglichen es, die Ausdrückwerkzeuge klein auszulegen. Dies hat zur Folge, daß die Außenabmessung des Implantats klein gewählt werden kann, weil die Ausnehmungen, durch welche die Ausdrückwerkzeuge geführt werden, klein gewählt werden können.

Erfindungsgemäß ist die Gleitschale aus Keramik hergestellt.

Nachfolgend wird die Erfindung anhand zweier Figuren näher erläutert.

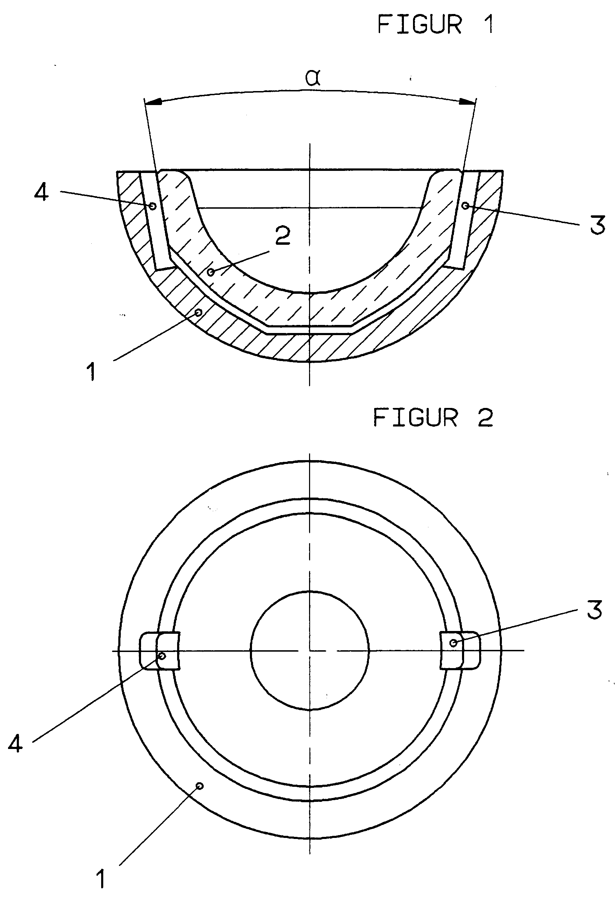

Figur 1 zeigt eine erfindungsgemäße Hüftgelenkpfanne im Schnitt und Figur 2 eine Metallschale in Draufsicht.

Die Implantataußenkontur bildet eine Metallschale 1. In diese Metallschale 1 ist eine Gleitschale 2 aus Keramik derart eingesetzt, daß die Oberkante der Metallschale 1 und der Gleitschale 2 aus Keramik auf derselben Höhe liegen. Die Gleitschale 2 ist mit Hilfe einer konischen Klemmung in der Metallschale fixiert. Erfindungsgemäß liegt der Winkel α der konischen Klemmung zwischen 10° und 25°, bevorzugt liegt er um 18°.

zum Einsetzen der Gleitschale 2 in die Metallschale 1 wird die Gleitschale 2 in die Metallschale 1 eingelegt und dann mittels eines Schlages, z.B. über einen Holzkeil, eingepreßt. Zum Heraushebeln der Gleitschale 2 sind erfindungsgemäß in der Metallschale 1 auf der Kontaktfläche zur Gleitschale 2 zwei diametral gegenüberliegende Ausnehmungen 3, 4 angeordnet. In diese Ausnehmungen 3, 4 wird jeweils ein Ausdrückwerkzeug (nicht gezeigt) eingeführt und gleichzeitig verdreht, so daß ein Druck von zwei Seiten von unten in Richtung der Auspressung ausgeübt wird.

Die erfindungsgemäße Hüftgelenkpfanne hat den Vorteil, daß die Gleitschale 2 fest in der Metallschale 1 verankert ist, sich jedoch leicht wieder entfernen läßt. Dies kann auch geschehen, wenn die Metallschale 1 schon implantiert ist, d.h. während einer Operation. Außerdem weist die Hüftgelenkpfanne eine kleine Baugröße auf.

## Patentansprüche

1. Hüftgelenkpfanne zum Einsetzen in Knochengewebe mit einer äußeren geschlossenen Metallschale (1) und einer inneren Gleitschale (2), wobei die Gleitschale (2) in der Metallschale (1) mit Hilfe einer konischen Klemmung fixiert ist, die Gleitschale (2) aus Keramik hergestellt ist, der Winkel (α) der konischen Klemmung zwischen 10° und 25° liegt und in der Metallschale (1) auf der Kontaktfläche zur Gleitschale (2) zumindest eine Ausnehmung (3) angeordnet ist, in die ein Ausdrückwerkzeug einführbar ist, mit dem ein Heraushebeln der Gleitschale (2) ermöglicht ist.

2. Hüftgelenkpfanne nach Anspruch 1, **dadurch gekennzeichnet**, daß der Winkel (α) der konischen Klemmung bei 18° liegt.

3. Hüftgelenkpfanne nach Anspruch 1 oder 2, **dadurch gekennzeichnet**, daß die zumindest eine Ausnehmung (3) bis unterhalb der konischen Klemmung reicht.

4. Hüftgelenkpfanne nach einem der Ansprüche 1 bis 3, **dadurch gekennzeichnet**, daß zwei Ausnehmungen (3, 4) diametral gegenüberliegend angeordnet sind.

5. Kombination einer Hüftgelenkpfanne nach einem der Ansprüche 1 bis 4 und einem Ausdrückwerkzeug, **dadurch gekennzeichnet**, daß das Ausdrückwerkzeug die Form eines Golfschlägers hat und durch Drehen des Ausdrückwerkzeugs eine Kraft von unten auf die Gleitschale (2) ausübbar ist.

## Claims

1. Hip-joint socket for insertion in osseous tissue, having an outer, closed metal shell (1) and an inner sliding shell (2), wherein the sliding shell (2) is fixed in the metal shell (1) with the aid of a conical clamping, the sliding shell (2) is made of ceramics, the angle (α) of the conical clamping is between 10° and 25°, and arranged in the metal shell (1), on the contact surface with respect to the sliding shell (2), is at least one recess (3), into which can be inserted an ejection tool, with which a levering-out of the sliding shell (2) is rendered possible.

2. Hip-joint socket according to claim 1, characterized in that the angle (α) of the conical clamping is 18°.

3. Hip-joint socket according to claim 1 or 2, characterized in that the at least one recess (3) extends until underneath the conical clamping.

4. Hip-joint socket according to one of claims 1 to 3, characterised in that two recesses (3, 4) are arranged in a manner such that they are diametrically opposed.

5. Combination of a hip-joint socket according to one of claims 1 to 4 and an ejection tool, characterised in that the ejection tool has the shape of a golf club and by rotating the ejection tool, a force can be exerted on the sliding shell (2) from underneath.

## Revendications

1. Coque acétabulaire pour l'implantation dans du tissu osseux, comprenant une cuvette métallique (1) extérieure fermée et un coussinet (2) intérieur, le coussinet (2) étant fixé dans la cuvette métallique (1) à l'aide d'une interface conique à serrage, le coussinet (2) étant en céramique, l'angle (α) de l'interface conique à serrage étant compris entre 10° et 25° et la cuvette métallique (1) présentant, à l'endroit de la surface de contact avec le coussinet (2), au moins un évidement (3) dans lequel peut être engagé un outil de décollement permettant de dégager le coussinet (2) par effet de levier.

2. Coque acétabulaire suivant la revendication 1, **caractérisée** par le fait que l'angle (α) de l'interface conique à serrage est de l'ordre de 18°.

3. Coque acétabulaire suivant la revendication 1 ou 2, **caractérisée** par le fait que l'évidement (3) s'étend jusqu'en dessous de l'interface conique à serrage.

4. Coque acétabulaire suivant l'une des revendications 1 à 3, **caractérisée** par le fait qu'il comprend deux évidements (3, 4) diamétralement opposés.

5. Combinaison d'une coque acétabulaire suivant l'une des revendications 1 à 4 et d'un outil de décollement, caractérisés par le fait que l'outil de décollement présente la forme d'un club de golf et que la rotation de l'outil de décollement permet d'exercer une force depuis le bas sur le coussinet (2).
